# EUROPÄISCHE PATENTSCHRIFT

(11) **EP 1 773 283 B1**
(45) Veröffentlichungstag und Bekanntmachung des Hinweises auf die Patenterteilung: **24.10.2007**
(21) Anmeldenummer: 05769191.7
(22) Anmeldetag: 18.06.2005
(51) Int. Cl.: A61Q 15/00, A61Q 17/02, A61Q 17/04, A61Q 19/00, A61Q 19/04, A61K 8/06, A61K 8/31, A61K 8/34, A61K 8/37, A61K 8/60, A61K 8/92

(54) **HOCHKONZENTRIERTE, SELBSTEMULGIERENDE EMULSIONSGRUNDLAGEN ZUR HERSTELLUNG VON ÖL IN WASSER EMULSIONEN**
HIGHLY CONCENTRATED SELF-EMULSIFYING EMULSION BASES USED FOR THE PRODUCTION OF OIL-IN-WATER EMULSIONS
BASES D'ÉMULSION AUTOÉMULSIFIANTES HAUTEMENT CONCENTRÉES SERVANT À PRODUIRE DES ÉMULSIONS HUILE DANS EAU

(30) Priorität: 29.06.2004 DE 102004031550
(43) Veröffentlichungstag der Anmeldung: 18.04.2007
(73) Patentinhaber: Cognis IP Management GmbH, 40589 Düsseldorf (DE)
(72) Erfinder: GOGET, Caroline, 40213 Düsseldorf (DE); ISSBERNER, Ulrich, 19002 Ambler (US); KAWA, Rolf, 40789 Monheim (DE); SORNS, Jörg, 40476 Düsseldorf (DE)
(86) Internationale Anmeldenummer: PCT/EP2005/006601
(87) Internationale Veröffentlichungsnummer: WO 2006/000360

(56) Entgegenhaltungen:
- EP-A- 1 247 519
- WO-A-02/43672
- WO-A-02/056841

## Beschreibung

### Gebiet der Erfindung

Gegenstand der Erfindung sind konzentrierte, transparente, bei 23°C fließfähige, selbstemulgierende Emulsionsgrundlagen/Konzentrate, die zur Herstellung von kosmetischen oder pharmazeutischen Emulsionen eingesetzt werden.

### Stand der Technik

Sogenannte selbstemulgierende Emulsionsgrundlagen zur Herstellung von Öl in Wasser Emulsionen enthalten aufeinander abgestimmte Mengen an Emulgatoren, Ölkomponenten und Wachsen. Es handelt sich hierbei üblicherweise um wasserfreie Produkte mit wachsartiger bis fester Konsistenz, deren Schmelzpunkte/Schmelzbereiche u.a. von den eingesetzten Wachsen und Ölkomponenten abhängt, die aber in der Regel Schmelzpunkte von mindestens 30°C aufweisen. Der Kosmetikchemiker erspart sich bei Einsatz derartiger Systeme zeitintensive Versuche bei der Entwicklung von kosmetischen Emulsionen. Gleichzeitig wird auch der Aufwand für Lagerhaltung Rohstoffprüfung beim Kunden reduziert. Selbstemulgierende Grundlagen auf Basis von Fettalkoholen und Alkyloligoglykosiden sind beispielsweise Gegenstand der EP 553 241 A1 und der WO 97/18033**.** Typische selbstemulgierende Grundlagen sind unter der Bezeichnung Emulgade® CL und Emulgade® CBN von der Fa. Cognis erhältlich. Zur Herstellung von Emulsionen werden in der Regel 10 - 20 Teile dieser Konzentrate (selbstemulgierenden Grundlagen), falls gewünscht mit weiteren Ölkörpern, auf eine Temperatur erhitzt, die 20°C über der Schmelztemperatur der selbstemulgierenden Grundlage liegt, und mit 80 Teilen Wasser, das ebenfalls auf diese Temperatur erhitzt wurde, emulgiert und ggf. mit weiteren Hilfsstoffen wie Konservierungsmittel, Parfum oder Wirkstoffen versetzt. Auf diese Weise entstehen Emulsionen mit Tröpfchen, die üblicherweise einen mittleren Teilchendurchmesser von ≥ 10 µm aufweisen. Um Sedimentationen zu vermeiden, sollte die Viskosität derartiger Emulsionen erfahrungsgemäß > 5000 mPa·s (Brookfield RVF, Spindel 5, 10 UpM b. 23°C) betragen. Die Einsatzmöglichkeit derartiger Konzentrate ist deutlich eingeschränkt. Sie sind nicht geeignet, wenn eine Verarbeitung in einem energiesparenden Kaltprozess vorgesehen ist oder wenn niedrigviskose Emulsionen mit einer Viskosität von ≤ 1000 mPa·s entwickelt werden sollen, die als Roll on, Spray oder als Tränkflüssigkeit für Wet-Wipe-Tücher Verwendung finden sollen.

Neben den sogenannten selbstemulgierenden Grundlagen werden im Markt auch Emulsionskonzentrate angeboten, die ebenfalls die wesentlichen Bestandteile einer Emulsion, nämlich Emulgatoren, Ölkomponenten und Wachse enthalten, und einen Wasseranteil von ca. 60% aufweisen. Diese Konzentrate werden üblicherweise nach dem PIT Verfahren hergestellt und weisen dementsprechend einen mittleren Tröpfchendurchmesser von < 1 µm, häufig zwischen 100 nm und 500 nm auf. Sie können problemlos mit Wasser kalt verdünnt werden und eignen sich besonders zur Herstellung von Roll-on-Konzepten, Sprays oder als Tränkflüssigkeit für Reinigungstücher. In Kombination mit viskositätsaufbauenden Polymeren können auch Lotionen und Cremes hergestellt werden. Nachteilig ist, dass die Zugabe von weiteren Ölkörpern nur unter Einsatz großer Homogenisierungsenergien möglich ist. Darüber hinaus sind derartige Emulsionskonzentrate aufgrund des hohen Wasseranteils mikrobiologisch anfällig und aus transport-ökonomischer Sicht ungünstig. Außerdem können derartige PIT-Konzentrate üblicherweise nur auf Basis von ethoxylierten Emulgatoren hergestellt werden. Diese Emulgatorklasse wird aber aus dermatologischen und ökologischen Gründen vermehrt negativ diskutiert. Sogennannte "ethoxylatfreie" Nanoemulsionen, allerdings mit relativ hohem Wassergehalt, sind aus der DE 103 46 515 bekannt, ethoxylatfreie selbstemulgierende Grundlagen aus der DE 103 47 940. O/W Emulsionen mit einem niedrigen Wassergehalt, die also prinzipiell Konzentraten entsprechen, sind auch aus der WO 02/056841 bekannt. Diese Emulsionen, die einen Wasseranteil von maximal 30% aufweisen, können allerdings den Nachteil aufweisen, dass sie bei Temperaturen > 30°C nicht lagerstabil sind und auch nicht ohne größeren Energieaufwand mit weiteren Ölkörpern emulgiert werden können.

Aufgabe der vorliegenden Erfindung war es daher, selbstemulgierende Emulsionsgrundlagen oder - konzentrate zur Verfügung zu stellen, die flüssig und dementsprechend kaltverarbeitbar sind, die einen niedrigen Wasseranteil aufweisen, problemlos mit weiteren Ölkörpern emulgiert werden können und mit Wasser weiter verdünnbar sind, ohne dass Separationen eintritt. Idealerweise liegen solche Konzentrate als transparente Lösung vor, da derartige Systeme im Gegensatz zu den herkömmlichen flüssigen Emulsionskonzentraten eine verbesserte Langzeitstabilität aufweisen.

### Beschreibung der Erfindung

Überraschenderweise wurde gefunden, dass eine Mischung enthaltend Laurylglucosid, Myristylglucosid, Polyglycerin-2-dipolyhydroxystearat, Cetylalkohol, Stearylalkohol und dünnflüssige Ölkörpern in genau aufeinander abgestimmten Verhältnissen mit maximal 10 Gew.-% Wasser die Aufgabenstellung lösen. Derartige Mischungen sind transparent, lassen sich problemlos mit weiteren Ölkörpern versetzen und mit Wasser verdünnen, ohne dass Separationen auftreten.

Gegenstand der vorliegenden Erfindung ist daher eine Zusammensetzung enthaltend:
(a) 3 - 5 Gew. % Laurylglucosid
(b) 1-2 Gew. % Myristylglucosid
(c) 5 - 7 Gew. % Polyglycerin-2-dipolyhydroxystearat
(d) 4 - 8 Gew.-% C12-C24-Fettalkohole
(e) 70 - 75 Gew. % Ölkörper
(f) 4 - 5 Gew.-% eines Hydrotrops ausgewählt aus den Diolen, Polyolen oder aus Glycerin und
(e) 2 -10 Gew. % Wasser.

Die Zusammensetzungen sind bei 23° C fließfähig, d.h. vorzugsweise weisen sie bei 23° C eine Viskosität von weniger als 2000 mPa·s auf (Brookfield RVF, Spindel 5,10 UpM). Vorzugsweise sind die Emulsionsgrundlagen transparent. In einer weiteren bevorzugten Ausführungsform haben sie eine mittlere Teilchengröße von maximal 100 nm, vorzugsweise von weniger als 100 nm vorzugsweise weniger als 50 nm und besonders bevorzugt weniger als 10 nm. Es handelt sich dabei um sogenannte micellare Lösungen.

In einer bevorzugten Ausführungsform sind die C12-C24 Fettalkohole ausgewählt aus Cetylalkohol oder Stearylalkohol oder einer Kombination dieser beiden Fettalkohole. In einer weiteren bevorzugten Ausführungsform ist das Hydrotrop Glycerin.

Eine besonders bevorzugte Ausführungsform enthält somit:
(a) 3 - 5 Gew. % Laurylglucosid
(b) 1-2 Gew. % Myristylglucosid
(c) 5-7 Gew. % Polyglycerin-2-dipolyhydroxystearat
(d) 4 - 8 Gew.-% Stearylalkohol oder Cetylalkohol oder eine beliebige Kombination dieser beiden Fettalkohole
(e) 70-75 Gew. % Ölkörper
(f) 4 - 5 Gew.-% Glycerin und
(g) 2 -10 Gew. % Wasser

Es hat sich gezeigt, dass nur Mischungen in dem angegebenen Verhältnis und auf Basis dieser Rohstoffe die geforderten Eigenschaften zeigen. Abweichungen führen dazu, dass die Mischungen eintrüben und sehr schnell separieren, nicht mehr mit weiteren Ölkörpern problemlos, das heißt ohne einen Homogenisierschritt, versetzt werden können und wässrige Verdünnungen sofort aufrahmen. Zu den bevorzugten Ölkörpern zählen wasserunlösliche, organische Verbindungen, die bei 25°C flüssig vorliegen und eine Viskosität von 1 bis 100 mPa·s (Höppler/Kugelfall-Methode, Deutsche Gesellschaft für Fettchemie, DGF C-IV 7, 20°C) aufweisen. Besonders bevorzugt sind dünnflüssige Ölkörper mit einer Viskosität von 1 bis 50 mPa·s.

### Gewerbliche Anwendbarkeit

Die erfindungsgemäßen Konzentrate können mit 30 - 95 Teilen Wasser pro 1 Teil Konzentrat verdünnt werden und zur Herstellung von Sprays, Roll-on-Konzepten, Wet-Wipe - Tränkflüssigkeiten und, unter Einsatz von Polymeren, zur Formulierung von Lotionen und / oder Cremes eingesetzt werden.

Ein weiterer Gegenstand der Erfindung sind daher kosmetische oder pharmazeutische Zubereitungen enthaltend 5-40 Gew.-% der erfindungsgemäßen Konzentrate. Vorzugsweise handelt es sich dabei um O/W-Emulsionen. Gegenstand der Erfindung ist auch die Verwendung der erfindungsgemäßen Emulsionskonzentrate zum Imprägnieren von Kosmetik-Tüchem und Wet Wipes sowie die Verwendung zur Herstellung von Körperpflegeformulierungen, Sonneschutzformulierungen, Antitranspirant-Formulierungen und Insektenschutz-Formulierungen. Ein weiterer Gegenstand der Anmeldung sind Wipes und Pads, die mit dem erfindungsgemäßen Emulsionskonzentrat oder einer Emulsion enthaltend 5 - 40 Gew.-% des Emulsionskonzentrates imprägniert sind. Aufgrund ihrer Feinteiligkeit und Transparenz eignen sich diese Emulsionskonzentrate und auch die daraus erhaltenen erfindungsgemäßen Emulsionen besonders gut zum Imprägnieren verschiedenartigster, saugfähiger Unterlagen. Beispiele für derartige beschichtete Substrate sind: Wipes zur Körperpflege und Intimhygiene, Abschminktücher, beschichtet Wattepads, Wipes mit Sonnenschutzformulierungen oder Insektenschutzformulierungen.

Ein weiterer Gegenstand der Anmeldung ist ein Verfahren zur Kalt-Herstellung von Emulsionen, wobei man das erfindungsgemäße Emulgatorkonzentrat mit einer Ölphase, die ggf. weitere öllösliche Komponenten enthält, und mit einer Wasserphase, enthaltend ggf. weitere wasserlösliche Wirkstoffe, versetzt und unter einfachem mechanischen Rühren dispergiert oder emulgiert, wobei der Emulgierungsprozess bei Temperaturen im Bereich von 15 - 40 °C, vorzugsweise 20 - 30 °C stattfindet. Hierbei ist es nicht notwendig Scherkräfte anzuwenden, einfaches mechanisches Rühren ist ausreichend. Die Viskosität kann, falls erforderlich, durch Zugabe von Polymeren eingestellt werden.

### Ölkörper/Ölkomponenten

Die erfindungsgemäßen kosmetischen oder pharmazeutischen Zubereitungen enthalten eine wässrige und eine Ölphase, die beide weitere Hilfs- und Zusatzstoffe enthalten können. Allerdings ist es aufgrund der Zusammensetzung des Emulgatorkonzentrates und seines hohen Ölanteils nicht zwingend notwendig, weitere Ölkomponenten zuzusetzen. Das Konzentrat kann auch nur mit eine wässrigen Phase verdünnt werden. Der Anteil der wässrigen Phase, inklusive wasserlöslicher Wirkstoffe, liegt üblicherweise im Bereich von 50 - 95 Gew.-% bezogen auf die Gesamtzusammensetzung, der Anteil der Ölphase bei 5 - 50 Gew.-% bezogen auf die Gesamtzusammensetzung des Endproduktes. Die Ölphase kann sich aus einer Ölkomponente oder einem beliebigen Gemisch von Ölkomponenten zusammensetzen sowie aus öllöslichen Wirkstoffen.

Als Ölkörper sind beispielsweise die nachstehend genannten Verbindungsklassen geeignet: Guerbetalkohole auf Basis von Fettalkoholen mit 6 bis 18, vorzugsweise 8 bis 10 Kohlenstoffatomen, z.B. 2-Ethylhexanol oder 2-Octyldodecanol; Ester von linearen oder verzweigten, gesättigten oder ungesättigten C₆-C₂₄-Fettsäuren mit linearen oder verzweigten, gesättigten oder ungesättigten C₆-C₂₄-Fettalkoholen. Beispielhaft seien genannt Hexyllaurat, Myristylisostearat, Myristyloleat, Cetylisostearat, Cetyloleat, Stearylisostearat, Stearyloleat, Isostearylmyristat, Isostearylpalmitat, Isostearylstearat, Isostearylisostearat, Isostearyloleat, Oleylmyristat, Oleylisostearat, Oleyloleat, Oleylerucat, Erucylisostearat, Erucyloleat, Cococaprylat/caprat. Weitere geeignete Ester sind z.B. Ester von C₁₈-C₃₈-Alkylhydroxycarbonsäuren mit linearen oder verzweigten, gesättigten oder ungesättigten C₆-C₂₂-Fettalkoholen, Ester von linearen und/oder verzweigten, gesättigten oder ungesättigten Fettsäuren mit mehrwertigen Alkoholen (wie z. B. Propylenglycol, Dimerdiol oder Trimertriol) und/oder Guerbetalkoholen, Triglyceride oder Triglyceridmischungen, flüssige Mono-/Di-/Triglyceridmischungen, Ester von C₆-C₂₂-Fettalkoholen und/oder Guerbetalkoholen mit aromatischen Carbonsäuren, insbesondere Benzoesäure (z. B. Finsolv® TN), Ester von C₂-C₁₂-Dicarbonsäuren mit linearen oder verzweigten, gesättigten oder ungesättigten Alkoholen mit 1 bis 22 Kohlenstoffatomen oder Polyolen mit 2 bis 10 Kohlenstoffatomen und 2 bis 6 Hydroxylgruppen. Geeignet sind auch pflanzliche Öle, Triglyceridmischungen, substituierte Cyclohexane, lineare symmetrische oder unsymmetrische Dialkylcarbonate (z.B. Cetiol® CC), Guerbetcarbonate auf Basis von Fettalkoholen mit 6 bis 18, vorzugsweise 8 bis 10 C-Atomen, lineare oder verzweigte, symmetrische oder unsymmetrische Dialkylether mit 6 bis 22 Kohlenstoffatomen pro Alkylgruppe, wie z. B. Di-n-octyl Ether (Cetiol® OE), Ringöffnungsprodukte von epoxidierten Fettsäureestern mit Polyolen, Kohlenwasserstoffe wie Paraffin- oder Mineralöle, Oligo- oder Polyalphaolefine. Die Dialkylcarbonate und Dialkylether können symmetrisch oder unsymmetrisch, verzweigt oder unverzweigt, gesättigt oder ungesättigt sein und lassen sich nach Reaktionen, die aus dem Stand der Technik hinlänglich bekannt sind, herstellen. Geeignete Siliconverbindungen sind beispielsweise Dimethylpolysiloxane, Methylphenylpolysiloxane, cyclische Silicone (Cyclomethicon) sowie amino-, fettsäure-, alkohol-, polyether-, epoxy-, fluor-, glykosid- und/oder alkylmodifizierte Siliconverbindungen. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt.

Erfindungsgemäß einsetzbar sind auch lineare Kohlenwasserstoffe mit einer Kettenlänge 8 bis 40 C-Atomen, die verzweigt oder unverzweigt, gesättigt oder ungesättigt sein können. Unter diesen sind verzweigte, gesättigte C8-C40-Alkane bevorzugt. Es können sowohl Reinsubstanzen eingesetzt werden als auch Substanzgemische. Üblicherweise handelt es sich um Substanzgemische verschiedener isomerer Verbindungen. Zusammensetzungen, die Alkane mit 10 bis 30, vorzugsweise 12 bis 20, und besonders bevorzugt 16 bis 20 Kohlenstoffatome aufweisen, sind besonders geeignet, und unter diesen ein Gemisch aus Alkanen, welches wenigstens 10 Gew.-% verzweigte Alkane bezogen auf die Gesamtmenge der Alkane enthält. Vorzugsweise handelt es sich um verzweigte, gesättigte Alkane. Besonders gut geeignet sind Gemische aus Alkanen ist, welche mehr als 1 Gew.-% 5,8-Diethyldodecan und/oder mehr als 1 Gew.-% Didecen enthalten.

### Weitere fakultative Hilfs- und Zusatzstoffe

Je nach Applikationszweck können die kosmetischen End-Formulierungen eine Reihe weiterer Hilfs- und Zusatzstoffe enthalten, wie beispielsweise Verdickungsmittel, Überfettungsmittel, Stabilisatoren, Polymere, Lecithine, Phospholipide, biogene Wirkstoffe, UV-Lichtschutzfaktoren, Antioxidantien, Deodorantien, Filmbildner, Quellmittel, Insektenrepellentien, Hydrotrope, Solubilisatoren, Konservierungsmittel, Parfümöle, Farbstoffe, die nachstehend exemplarisch aufgelistet sind. Ggf. könnten auch noch die weiteren üblichen Emulgatoren zugesetzt werden, obgleich dies nicht notwendig ist. Die Mengen der jeweiligen Zusätze richten sich nach der beabsichtigten Verwendung.

Als **Verdickungsmittel** eignen sich beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethyl- und Hydroxypropylcellulose, Polyvinylalkohol, Polyvinylpyrrolidon und Bentonite wie z. B. Bentone^{®} Gel VS-5PC (Rheox).

Unter oder pharmazeutische sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z. B. Wärme wieder abzugeben. UV-B-Filter können öllöslich oder wasserlöslich sein. Als typische UV-A-Filter kommen insbesondere Derivate des Benzoylmethans in Frage. Die UV-A und UV-B-Filter können selbstverständlich auch in Mischungen eingesetzt werden, z.B. Kombinationen aus den Derivaten des Benzoylmethans, z. B. 4-tert.-Butyl-4'-methoxydibenzoylmethan (Parsol^{®} 1789) und 2-Cyano-3,3-phenylzimtsäure-2-ethyl-hexylester (Octocrylene) sowie Estern der Zimtsäure, vorzugsweise 4-Methoxyzimtsäure-2-ethylhexylester und/oder 4-Methoxyzimtsäurepropylester und/oder 4-Methoxyzimtsäureisoamylester. Häufig werden derartige Kombinationen mit wasserlöslichen Filtern wie z.B. 2-Phenylbenzimidazol-5-sulfonsäure und deren Alkali-, Erdalkali-, Ammonium-, Alkylammonium-, Alkanolammonium- und Glucammoniumsalze kombiniert.

Neben den genannten löslichen Stoffen kommen auch unlösliche Lichtschutzpigmente, nämlich feindisperse Metalloxide in Frage. Beispiele für geeignete Metalloxide sind insbesondere Zinkoxid und Titandioxid. Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt.

Unter oder pharmazeutische sind beispielsweise Tocopherol, Tocopherolacetat, Tocopherolpalmitat, Ascorbinsäure, (Desoxy)Ribonucleinsäure und deren Fragmentierungsprodukte, β-Glucane, Retinol, Bisabolol, Allantoin, Phytantriol, Panthenol, AHA-Säuren, Aminosäuren, Ceramide, Pseudoceramide, essentielle Öle, Pflanzenextrakte, wie z. B. Prunusextrakt, Bambaranussextrakt und Vitaminkomplexe zu verstehen.

**Desodorierende Wirkstoffe** wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend eignen sich als deosodorierende Wirkstoffe u.a. keimhemmende Mittel, Enzyminhibitoren, Geruchsabsorber oder Geruchsüberdecker.

Als **Insekten-Repellentien** kommen beispielsweise N,N-Diethyl-m-toluamid, 1,2-Pentandiol oder 3-(N-n-Butyl-N-acetyl-amino)-propionsäureethylester), welches unter der Bezeichnung Insect Repellent^{®} 3535 von der Merck KGaA vertrieben wird, sowie Butylacetylaminopropionate in Frage.

Als **Selbstbräuner** eignet sich Dihydroxyaceton. Als Tyrosinhinbitoren, die die Bildung von Melanin verhindern und Anwendung in Depigmentierungsmitteln finden, kommen beispielsweise Arbutin, Ferulasäure, Kojisäure, Cumarinsäure und Ascorbinsäure (Vitamin C) in Frage.

Als **Konservierungsmittel** eignen sich beispielsweise Phenoxyethanol, Formaldehydlösung, Parabene, Pentandiol oder Sorbinsäure sowie die unter der Bezeichnung Surfacine® bekannten Silberkomplexe und die in Anlage 6, Teil A und B der Kosmetikverordnung aufgeführten weiteren Stoffklassen.

Als **Parfümöle** seien genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen, Harzen und Balsamen. Weiterhin kommen tierische Rohstoffe, wie beispielsweise Zibet und Castoreum sowie synthetische Riechstoffverbindungen vom Typ der Ester, Ether, Aldehyde, Ketone, Alkohole und Kohlenwasserstoffe in Frage.

Als **Farbstoffe** können die für kosmetische Zwecke geeigneten und zugelassenen Substanzen verwendet werden. Beispiele sind Kochenillerot A (C.I. 16255), Patentblau V (C.I.42051), Indigotin (C.I.73015), Chlorophyllin (C.I.75810), Chinolingelb (C.I.47005), Titandioxid (C.I.77891), Indanthrenblau RS (C.I. 69800) und Krapplack (C.I.58000). Diese Farbstoffe werden üblicherweise in Konzentrationen von 0,001 bis 0,1 Gew.-%, bezogen auf die gesamte Mischung, eingesetzt.

Die nachfolgenden Beispiele betreffen die erfindungsgemäßen Emulgatorkonzentrate und Emulsionen, die auf Basis der erfindungsgemäßen Emulgatorzusammensetzungen hergestellt wurden.

### Beispiele

Rezepturen 1 - 4 in der Tabelle 1 geben erfindungsgemäße Beispiele wieder. Rezepturen V1 - V3, die außerhalb der erfindungsgemäßen Grenzen liegen, dienen zum Vergleich. Die Mengenangaben beziehen sich auf Gew.-% der Gesamtzusammensetzung.

**Tabelle 1**

| **Inhaltsstoffe** | **1** | **2** | **3** | **4** | **V1** | **V2** | **V3** |
|---|---|---|---|---|---|---|---|
| Laurylglucosid | 4,2 | 4,6 | 3,8 | 4,9 | 2,8 | 4,2 | 5,3 |
| Myristylglucosid | 1,7 | 1,4 | 1,3 | 1,8 | 1,2 | 1,7 | - |
| Polyglycerin-2-di-polyhydroxystearat | 6,0 | 6,0 | 5,4 | 6,4 | 6,0 | 6,0 | 5,3 |
| Myristylalkohol | - | - | - | - | - | 3,5 | - |
| Cetylalkohol | 2,2 | 2,5 | 6,0 | - | 2,2 | - | - |
| Stearylalkohol | 2,3 | 2,5 | - | 6,0 | 2,3 | - | - |
| Glycerylstearat | - | - | - | - | - | - | 3,0 |
| Cocoglyceride | 36,0 | 30,0 | - | - | 36,0 | 36,0 | 30,0 |
| Dicaprylylcarbonat | 38,0 | 12,0 | - | - | 38,0 | 38,0 | 30,0 |
| Octyldodecanol | - | - | 30,0 | - | - | - | - |
| Cetearylisononanoat | - | 15,0 | - | 32,0 | - | - | - |
| Caprylic/Capric Triglyceride | - | 15,0 | 30,0 | - | - | - | - |
| Isopropylmyristat | - | - | - | 25,0 | - | - | - |
| Mineral Oil | - | - | 13,0 | 13,0 | - | - | - |
| Glycerin | 4,5 | 4,5 | 4,5 | 4,5 | 4,5 | 4,5 | 4,0 |
| Wasser | 5,1 | 6,5 | 6,0 | 6,4 | 7,0 | 6,1 | 22,4 |
| | | | | | | | |
| Aussehen | Transparent | transparent | transparent | transparent | trüb | transparent | trüb |
| Phasenstabilität 4 Wochen RT/40°C | + | + | + | + | - | + | - |
| Verdünnbar mit H₂O | homogen | homogen | homogen | homogen | Separation | Separation | ja |
| Verdünnbar mit Öl | ja | ja | ja | ja | ja | Ja | Separation |

Die Ergebnisse zeigen, dass nur die erfindungsgemäßen Beispiele phasenstabil sind und/oder problemlos weiterverarbeitet werden können.

Die nachfolgenden Beispiele beschreiben die technische Anwendung der erfindungsgemäßen Konzentrate. Die Mengenangaben beziehen sich auf Gew.-% der Gesamtzusammensetzung.

### 1. Anwendung für Wet Wipe Konzepte

Die Tücher können besprüht oder aber auch getränkt werden. Es wurden je ca. 3 g der nachfolgenden Emulsionen (Beispiel 1a,1b) pro 1 g des Tuches aufgetragen. Tuchmaterial: Spunlace Viscose 65% / Polyester 35% - 55 g/m².

| | |
|---|---|
| 1a. Konzentrat **Beispiel 1** | 10,0 Gew.-% |
| Wasser, Konservierungsmittel | 90,0 Gew.-% |
| | |
| 1b. Konzentrat **Beispiel 1** | 8,0 Gew.-% |
| Cetearylisononanoat | 4,0 Gew.-% |
| Wasser, Konservierungsmittel | 88,0 Gew.-% |

### 2. Anwendung für Hautpflegekonzepte

| 2a. Bodyspray | |
|---|---|
| Konzentrat **Beispiel 2** | 12,0 Gew.-% |
| Wasser, Konservierungsmittel | 88,0 Gew.-% |
| | |

| 2b. Bodylotion | |
|---|---|
| Konzentrat **Beispiel 4** | 10,0 Gew.-% |
| Dicaprylylcarbonat | 10,0 Gew.-% |
| Vitamin E | 2,0 Gew.-% |
| Natriumpolyacrylat | 0,7 Gew.-% |
| Wasser, Konservierungsmittel | 77,3 Gew.-% |
| | |

| 2c. Sonnenspray | |
|---|---|
| Konzentrat Beispiel 1 | 15,0 Gew.-% |
| Dicaprylylcarbonat | 5,0 Gew.-% |
| Cosmedia® DC | 2,0 Gew.-% |
| Ethylhexylmethoxycinnamat | 7,5 Gew.-% |
| Butylmethoxydibenzoylmethan | 2,5 Gew.-% |
| Natriumpolyacrylat | 0,1 Gew.-% |
| Wasser, Konservierungsmittel | 67,9 Gew.-% |
| | |

| 2d. Selbstbräunungsspray | |
|---|---|
| Konzentrat **Beispiel 4** | 10,0 Gew.-% |
| Dicaprylylcarbonat | 10,0 Gew.-% |
| Vitamin E | 2,0 Gew.-% |
| Natriumpolyacrylat | 0,7 Gew.-% |
| Dihydroxyaceton | 4,0 Gew.-% |
| Wasser, Konservierungsmittel | 73,3 Gew.-% |
| | |

| 2e. Insektrepellent Spray | |
|---|---|
| Konzentrat **Beispiel 1** | 10,0 Gew.-% |
| Dicaprylylcarbonat | 10,0 Gew.-% |
| Vitamin E | 2,0 Gew.-% |
| Natriumpolyacrylat | 0,7 Gew.-% |
| Ethylbutylacetylaminopropionat | 8,0 Gew.-% |
| Wasser, Konservierungsmittel | 69,3 Gew.-% |

### 3. Anwendung für AP/Deo Konzepte

| 3a. AP/Deo Spray | |
|---|---|
| Konzentrat **Beispiel 3** | 10,0 Gew.-% |
| Dicaprylylcarbonat | 10,0 Gew.-% |
| Triethylcitrat | 2,0 Gew.-% |
| Vitamin E | 2,0 Gew.-% |
| Aluminiumchlorohydrat | 6,0 Gew.-% |
| Wasser, Konservierungsmittel | 70,0 Gew.-% |
| | |

| 3b. AP/Deo Roll on | |
|---|---|
| Konzentrat Beispiel 3 | 10,0 Gew.-% |
| Dicaprylylcarbonat | 10,0 Gew.-% |
| Triethylcitrat | 2,0 Gew.-% |
| Vitamin E | 2,0 Gew.-% |
| Hydroxypropylcellulose | 0,5 Gew.-% |
| Aluminiumchlorohydrat | 6,0 Gew.-% |
| Wasser, Konservierungsmittel | 69,5 Gew.-% |
| | |

| 3c. AP/Deo Roll on | |
|---|---|
| Konzentrat Rezeptur 3 | 15 Gew.-% |
| Cyclomethicon | 10 Gew.-% |
| Triethylcitrat | 4 Gew.-% |
| Vitamin E | 1 Gew.-% |
| Hydroxypropylcellulose | 0,5 Gew.-% |
| Aluminium-Zirkoniumtetra-chlorhydrat | 10 Gew.-% |
| Wasser, Konservierungsmittel | 59,5 Gew.-% |

In den aufgeführten Beispielen wurden als Konservierungsmittel Phenonip, Euxyl K 702 und Symdiol 68 eingesetzt.

### 4. Anwendung für Körperpflegekonzepte

| **4a. Körpercreme** | |
|---|---|
| Konzentrat Rezeptur 4 | 25 Gew.-% |
| Dicaprylylether | 5 Gew.-% |
| Natriumpolyacrylat | 1,5 Gew.-% |
| Aluminiumstarchoctenylsuccinat | 1 Gew.-% |
| Glycerin | 3 Gew.-% |
| Wasser, Konservierung | 64,5 Gew.-% |
| | |

| **4b. Körperlotion** | |
|---|---|
| Konzentrat Rezeptur 4 | 20 Gew.-% |
| Cyclomethicon | 4 Gew.-% |
| Cosmedia DC | 1,5 Gew.-% |
| (Hydrogenated Dimer Dilinoleyl/Dimethylcarbonate Copolymer) | |
| Carbomer | 0,5 Gew.-% |
| Glycerin | 2 Gew.-% |
| Wasser, Konservierung | 72 Gew.-% |
| | |

| **4c. Körperlotion** | |
|---|---|
| Konzentrat Rezeptur 4 | 20 Gew.-% |
| Hydriertes Polyisobuten | 4 Gew.-% |
| Carbomer | 0,5 Gew.-% |
| Glycerin | 3 Gew.-% |
| Wasser, Konservierung | 72,5 Gew.-% |

### 5. Anwendung für Sonnenschutzkonzepte

| **5a. Sonnenschutzlotion** | |
|---|---|
| Konzentrat Rezeptur 1 | 25 Gew.-% |
| Titandioxid nanonisiert | 3 Gew.-% |
| Zinkoxid nanonisiert | 4 Gew.-% |
| Cosmedia DC | 1 Gew.-% |
| Glycerin | 3 Gew.-% |
| Wasser, Konservierung | 64 Gew.-% |
| | |

| **5b. Sonnenschutzcreme** | |
|---|---|
| Konzentrat Rezeptur 2 | 25 Gew.-% |
| Dibutyladipat | 5 Gew.-% |
| Ethylhexyltriazon | 2 Gew.-% (Uvinul T 150) |
| Diethylamino-hydroxy-benzoylhexylbenzoat | 2 Gew.-% (Uvinul A plus) |
| Ethylhexylmethoxycinnamat | 6 Gew.-% (Neo Heliopan AV) |
| Cosmedia DC | 1 Gew.-% |
| (Hydrogenated Dimer Dilinoleyl / Dimethylcarbonate Copolymer) | |
| Natriumpolyacrylat | 1 Gew.-% |
| Glycerin | 3 Gew.-% |
| Wasser, Konservierung | 55 Gew.-% |
| | |

| **5c. Sonnenschutzlotion** | |
|---|---|
| Konzentrat Rezeptur 1 | 25 Gew.-% |
| Dibutyladipat | 3 Gew.-% |
| Diethylhexylbutamidotriazon | 2 Gew.-% (Uvasorb HEB) |
| Ethylhexylmethoxycinnamate | 6 Gew.-% (Neo Heliopan AV) |
| Natriumphenylbenzimidazolsulfonat | 2 Gew.-% (Neo Heliopan hydro) |
| Dinatriumphenyldibenzimidazoltetrasulfonat | 2 Gew.-% (Neo Heliopan AP) |
| Cosmedia DC | 1 Gew.-% |
| (Hydrogenated Dimer Dilinoleyl / Dimethylcarbonate Copolymer) | |
| Xanthan Gum | 0,5 Gew.-% |
| Glycerin | 3 Gew.-% |
| Wasser, Konservierung | 55,5 Gew.-% |
| | |

| **5d. Sonnenschutzlotion** | |
|---|---|
| Konzentrat Rezeptur 1 | 25 Gew.-% |
| Dibutyladipat | 5 Gew.-% |
| Methylen-bis-benzotriazolyl tetramethylbutylphenol | 2 Gew.-% (Tinosorb M) |
| Bis-ethylhexyloxyphenolmethoxyphenyltriazin | 2 Gew.-% (Tinosorb S) |
| Ethylhexylmethoxycinnamate | 6 Gew.-% (Neoo Heliopan AV) |
| Natriumphenylbenzimidazolsulfonat | 2 Gew.-% (Neo Heliopan hydro) |
| Dinatriumphenyldibenzimidazoltetrasulfonat | 2 Gew.-% (Neo Heliopan AP) |
| Cosmedia DC | 1 Gew.-% |
| (Hydrogenated Dimer Dilinoleyl / Dimethylcarbonate Copolymer) | |
| Xanthan Gum | 0,5 Gew.-% |
| Glycerin | 3 Gew.-% |
| Wasser, Konservierung | 51,5 Gew.-% |
| | |

| **5e. Sonnenschutzcreme** | |
|---|---|
| Konzentrat Rezeptur 2 | 25 Gew.-% |
| Dibutyladipat | 5 Gew.-% |
| Octocrylen | 2 Gew.-% |
| Methylbenzylidencampher | 2 Gew.-% (Neo Heliopan MBC) |
| Ethylhexylmethoxycinnamat | 6 Gew.-% (Neo Heliopan AV) |
| Cosmedia DC | 1 Gew.-% |
| (Hydrogenated Dimer Dilinoleyl / Dimethylcarbonate Copolymer) | |
| Natriumpolyacrylat | 1,2 Gew.-% |
| Glycerin | 3 Gew.-% |
| Wasser, Konservierung | 54,8 Gew.-% |

### 6. Anwendung für Insektenschutzkonzepte

| **6a. Insectrepellent Lotion** | |
|---|---|
| Konzentrat Rezeptur 4 | 25 Gew.-% |
| Dicaprylylether | 5 Gew.-% |
| Natriumpolyacrylat | 0,5 Gew.-% |
| N,N-Diethyl-m-toluolamid | 2 Gew.-% |
| Glycerin | 3 Gew.-% |
| Wasser, Konservierung | 64,5 Gew.-% |

## Patentansprüche

1. Emulsionskonzentrat enthaltend:
(a) 3-5 Gew. % Laurylglucosid
(b) 1-2 Gew. % Myristylglucosid
(c) 5-7 Gew. % Polyglycerin-2-dipolyhydroxystearät
(d) 4 - 8 Gew.% C12-C24-Fettalkohole
(e) 70 - 75 Gew. % Ölkörper
(f) 4 - 5 Gew.-% eines Hydrotrops ausgewählt aus den Diolen, Polyolen oder aus Glycerin und
(g) 2 -10 Gew. % Wasser.

2. Emulsionskonzentrat gemäß Anspruch 1, **dadurch gekennzeichnet, dass** es bei 23° C eine Viskosität von weniger als 2000 mPa·s aufweist, gemessen mit einem Brookfield RVF Viskosimeter, mit Spindel 5, bei 10 Umdrehungen pro Minute.

3. Emulsionskonzentrat gemäß wenigstens einem der Ansprüche 1 bis 2, **dadurch gekennzeichnet dass** es transparent ist.

4. Emulsionskonzentrat gemäß wenigstens einem der Ansprüche 1 bis 3, **dadurch gekennzeichnet, dass** es eine mittlere Tröpfchengröße von maximal 100 nm, vorzugsweise weniger als 100 nm, vorzugsweise weniger als 50 nm aufweist.

5. Kosmetische oder pharmazeutische Zusammensetzung enthaltend 5 - 40 Gew.% eines Emulsionskonzentrates gemäß einem der Ansprüche 1 bis 4.

6. Verwendung des Emulsionskonzentrates gemäß einem der Ansprüche 1 bis 4 zur Herstellung von Körperpflegeformulierungen, Sonneschutzformulierungen, Antitranspirant-Formulierungen, Insektenschutz-Formulierungen und zur Beschichtung von Kosmetik-Tüchem.

7. Verfahren zur Kalt-Herstellung von Emulsionen, wobei man das Emulsionskonzentrat gemäß wenigstens einem der Ansprüche 1 bis 4 mit einer Ölphase, die ggf. weitere öllösliche Komponenten enthält, und mit einer Wasserphase, enthaltend ggf. weitere wasserlösliche Wirkstoffe, versetzt und unter einfachem mechanischen Rühren dispergiert oder emulgiert, wobei der Emulgierungsprozess bei Temperaturen im Bereich von 15 - 40 °C, vorzugsweise 20 - 30 °C stattfindet.

8. Wipes und Pads, die mit einem Emulsionskonzentrat gemäß einem der Ansprüche 1 bis 4 oder einer Emulsion nach Anspruch 5 imprägniert sind.

## Claims

1. Emulsion concentrate containing:
(a) 3 to 5% by weight lauryl glucoside,
(b) 1 to 2% by weight myristyl glucoside,
(c) 5 to 7% by weight polyglycerol-2-dipolyhydroxystearate,
(d) 4 to 8% by weight C₁₂₋₂₄ fatty alcohols,
(e) 70 to 75% by weight oil components,
(f) 4 to 5% by weight of a hydrotrope selected from diols, polyols or from glycerol and
(g) 2 to 10% by weight water.

2. Emulsion concentrate as claimed in claim 1, **characterized in that** it has a viscosity at 23°C of less than 2,000 mPa.s, as measured with a Brookfield RVF viscosimeter, spindle 5,10 r.p.m.

3. Emulsion concentrate as claimed in at least one of claims 1 to 2, **characterized in that** it is transparent.

4. Emulsion concentrate as claimed in at least one of claims 1 to 3, **characterized in that** it has a mean droplet size of at the most 100 nm, preferably less than 100 nm and preferably less than 50 nm.

5. Cosmetic or pharmaceutical composition containing 5 to 40% by weight of the emulsion concentrate claimed in any of claims 1 to 4.

6. Use of the emulsion concentrate claimed in any of claims 1 to 4 for the production of body care formulations, sun protection formulations, antiperspirant formulations, insect repellent formulations and for coating cosmetic wipes.

7. Process for the cold production of emulsions in which an oil phase optionally containing other oil-soluble components and a water phase optionally containing other water-soluble active components are added to the emulsion concentrate claimed in at least one of claims 1 to 4 and dispersed or emulsified therein with simple mechanical stirring, the emulsifying process taking place at temperatures of 15 to 40°C and preferably at temperatures of 20 to 30°C.

8. Wipes and pads impregnated with the emulsion concentrate claimed in any of claims 1 to 4 or with the emulsion claimed in claim 5.

## Revendications

1. Concentré d'émulsion contenant :
(a) de 3 à 5 % en poids de glucoside de lauryle
(b) de 1 à 2 % en poids de glucoside de myristyle
(c) de 5 à 7 % en poids de 2-dipolyhydroxystéarate de polyglycérol
(d) de 4 à 8 % en poids d'alcools gras en C 12 à C24,
(e) de 70 à 75 % en poids de corps huileux
(f) de 4 à 5 % en poids d'un agent hydrotrope choisi parmi les diols, les polyols ou le glycérol, et
(g) de 2 à 10 % en poids d'eau.

2. Concentré d'émulsion selon la revendication 1,
**caractérisé en ce qu'**
il a une viscosité inférieure à 2000 mPa·s à 23°C mesurée avec un viscosimètre RVF de Brookfield, avec une broche de 5 et à 10 tours par minute.

3. Concentré d'émulsion selon au moins l'une des revendications 1 et 2,
**caractérisé en ce qu'**
il est transparent.

4. Concentré d'émulsion selon au moins l'une des revendications 1 à 3,
**caractérisé en ce qu'**
il présente une taille moyenne des gouttelettes d'au maximum 100 nm, de préférence inférieure à 100 nm, de préférence inférieure à 50 nm.

5. Composition cosmétique ou pharmaceutique, contenant de 5 à 40 % en poids d'un concentré d'émulsion selon l'une des revendications 1 à 4.

6. Utilisation du concentré d'émulsion selon l'une des revendications 1 à 4, pour préparer des formulations de soin du corps, des formulations de protection solaire, des formulations d'antitranspirants, des formula tions de protection contre les insectes et pour le revêtement de lingettes cosmétiques.

7. Procédé de préparation à froid d'émulsions,
selon lequel on mélange le concentré d'émulsion selon au moins l'une des revendications 1 à 4, avec une phase huileuse contenant le cas échéant d'autres composants solubles dans l'huile et une phase aqueuse contenant le cas échéant d'autres agents actifs hydrosolubles, et on disperse ou émulsionne ce mélange sous agitation mécanique simple, le procédé d'émulsification étant mis en oeuvre à des températures de l'ordre de 15°C à 40°C, de préférence de 20°C à 30°C.

8. Lingettes et disques imprégnés d'un concentré d'émulsion selon l'une des revendications 1 à 4 ou d'une émulsion selon la revendication 5.
